# EUROPEAN PATENT SPECIFICATION

(11) **EP 2 823 302 B1**
(45) Date of publication and mention of the grant of the patent: **10.05.2017**
(21) Application number: 13720562.1
(22) Date of filing: 07.03.2013
(51) Int. Cl.: G01N 33/50

(54) **PROCESS AND DEVICE FOR THE DETERMINATION OF ALTERATIONS IN NEURONAL CONNECTIVITY AND/OR MORPHOLOGY**
VERFAHREN UND VORRICHTUNG ZUR FESTSTELLUNG VON ÄNDERUNGEN DER NEURONALEN KONNEKTIVITÄT UND/ODER MORPHOLOGIE
PROCESSUS ET DISPOSITIF DE DÉTERMINATION DE MODIFICATIONS DE CONNECTIVITÉ ET / OU DE MORPHOLOGIE NEURONALE

(30) Priority: 07.03.2012 IT TO20120197
(43) Date of publication of application: 14.01.2015
(73) Proprietor: Fondazione Istituto Italiano Di Tecnologia, 16163 Genova (IT)
(72) Inventor: BERDONDINI, Luca, I-16128 Genova (IT); DANTE, Silvia, I-17047 Vado Ligure (Savona) (IT); MARCONI, Emanuele, I-16163 Genova (IT); SALERNO, Marco, I-19025 La Spezia (IT); DE PIETRI TONELLI, Davide, I-16011 Arenzano (Genova) (IT)
(74) Representative: Rambelli, Paolo
(86) International application number: PCT/IB2013/051804
(87) International publication number: WO 2013/132455

(56) References cited:
- TOUROVSKAIA A ET AL: "Micropatterns of chemisorbed cell adhesion-repellent films using oxygen plasma etching and elastomeric masks", LANGMUIR, AMERICAN CHEMICAL SOCIETY, NEW YORK, NY; US, vol. 19, no. 11, 27 May 2003 (2003-05-27), pages 4754-4764, XP002367767, ISSN: 0743-7463, DOI: 10.1021/LA0267948
- LI ET AL: "Integration of topographical and biochemical cues by axons during growth on microfabricated 3-D substrates", EXPERIMENTAL CELL RESEARCH, ACADEMIC PRESS, US, vol. 311, no. 2, 10 December 2005 (2005-12-10), pages 307-316, XP005166109, ISSN: 0014-4827, DOI: 10.1016/J.YEXCR.2005.10.007
- YONG HEE KIM ET AL: "Enhancement of neuronal cell adhesion by covalent binding of poly--lysine", JOURNAL OF NEUROSCIENCE METHODS, ELSEVIER SCIENCE PUBLISHER B.V., AMSTERDAM, NL, vol. 202, no. 1, 23 August 2011 (2011-08-23), pages 38-44, XP028308287, ISSN: 0165-0270, DOI: 10.1016/J.JNEUMETH.2011.08.036 [retrieved on 2011-08-31]
- MESSA M ET AL: "Growth Cone 3-D Morphology is Modified by Distinct Micropatterned Adhesion Substrates", IEEE TRANSACTIONS ON NANOBIOSCIENCE, IEEE SERVICE CENTER, PISCATAWAY, NY, US, vol. 8, no. 2, 1 June 2009 (2009-06-01), pages 161-168, XP011268211, ISSN: 1536-1241, DOI: 10.1109/TNB.2009.2019109
- MARCO SALERNO ET AL: "AFM measurement of the stiffness of layers of agarose gel patterned with polylysine", MICROSCOPY RESEARCH AND TECHNIQUE, 1 January 2010 (2010-01-01), pages NA-NA, XP055038833, ISSN: 1059-910X, DOI: 10.1002/jemt.20838
- SALERNO M ET AL: "Local increase in stiffness of agarose gel layer by patterning with polylysine measured via atomic force microscopy", JOURNAL OF THE MECHANICAL BEHAVIOR OF BIOMEDICAL MATERIALS, ELSEVIER, AMSTERDAM, NL, vol. 3, no. 6, 1 April 2010 (2010-04-01), pages 425-435, XP027113308, ISSN: 1751-6161 [retrieved on 2010-06-29]
- DOWELL-MESFIN N M ET AL: "Topographically modified surfaces affect orientation and growth of hippocampal neurons; Topographically modified surfaces affect orientation and growth of hippocampal neurons", JOURNAL OF NEURAL ENGINEERING, INSTITUTE OF PHYSICS PUBLISHING, BRISTOL, GB, vol. 1, no. 2, 1 June 2004 (2004-06-01), pages 78-90, XP020082864, ISSN: 1741-2552, DOI: 10.1088/1741-2560/1/2/003
- EMANUELE MARCONI ET AL: "Emergent Functional Properties of Neuronal Networks with Controlled Topology", PLOS ONE, vol. 7, no. 4, 6 April 2012 (2012-04-06), page e34648, XP055038830, DOI: 10.1371/journal.pone.0034648
- YUZO TAKAYAMA ET AL: "Formation of one-way-structured cultured neuronal networks in microfluidic devices combining with micropatterning techniques", JOURNAL OF BIOSCIENCE AND BIOENGINEERING, vol. 114, no. 1, 24 April 2012 (2012-04-24), pages 92-95, XP055038903, ISSN: 1389-1723, DOI: 10.1016/j.jbiosc.2012.02.011

## Description

The present invention relates to a bioassay process and to a device for carrying out the assay for detecting and/or quantifying the ability of neuronal cells to form neuritic extensions and/or to connect other neurons.

The process and the device which are the subject of the invention are useful in particular as a research tool applied to animal models for the study of the functional properties of neuronal cells, and also as a tool in the diagnosis of neuronal or neurodegenerative diseases associated with undesired alterations in the neuronal connectivity and/or morphology on cells from animal models or non-embryonic human cells.

In the research concerning the physiological status of neuronal cells in their interactions/correlations, that is in their electrical communication through the axons and the terminal growth cones, the importance of being able to have available neuronal cells in well-defined positions such as for example in a lattice, and of the possibility of exerting a dominant influence on the respective connections thereof, is known.

The techniques for standard patterning of materials are based on photolithography and electron beam lithography techniques typical of the microelectronics industry, taking advantage of the relevant technology developed in the last 50 years.

However, these techniques are difficult to combine with organic and biological, sensitive and delicate materials typical of the biosciences, in that they are applied to rigid organic materials (silicon wafer) and are based on electromagnetic rays which cause damage and involve the use of aggressive organic solvents during the process stages.

Alternatively to the techniques of radiation lithography, the so-called techniques of soft lithography which make it possible to obtain models of cellular adhesion/repulsion signals, according to any desired arrangement, are also known.

These techniques are generally based on the imprinting of a defined stamp (master), constituting a model, onto a substrate of interest for cell growth, by means of an "ink" of organic/biological/nanoparticle interest.

However, in spite of their name, these recent techniques are also based on the standard methods of lithography (based on radiation) mentioned above, for the fabrication of the aforesaid stamp, which is usually effected on masks of silicon or rigid quartz wafer and only subsequently reproduced if necessary in a "soft" replica (usually of PDMS).

Thus the preparation of parallel soft lithography patterns is equally expensive, costly in terms of time, and requires equipment of large dimensions and high cost owing to the necessity to create the aforesaid stamp, and is therefore not readily adaptable to the rapid production of prototypes which may be required when the initial properties of the pattern in question are not well defined.

Moreover, the techniques for standard patterning do not permit a morphological neuronal bioassay, or indeed a quantification of neuritic extension and the ability of the neurons to interconnect. Messa et al., 2009 deals with sensing properties of neurons. It discloses a method and device comprising a grid of an adhesion-promoting substance, such as L1-Fc, on an adhesion-inhibiting surface, such as glass. Neuronal cells are applied to the surface and growth is determined by immunofluorescence. The present invention solves the problem of creating networks of neuronal cells well characterized in terms of behaviour with regard to their morphology and their topology. Furthermore, the invention solves the problem of providing a technique for the production of patterns of neuronal cells based on soft organic materials on simple physical (mechanical) contact methodologies with the use of an immersion nib tip.

It has also been demonstrated that the technique for preparation of the pattern used in the invention is able to decouple the topographical (morphological) character of the pattern from the physico-chemical properties (i.e. chemical affinity, contact rigidity, electrical charge or surface electrical potential).

Another purpose of the invention is that of providing an assay which simplifies the morphological evaluation of the neuronal branching, currently difficult and costly in terms of time, since it is based on visualization techniques and subsequent image analysis, such as the Sholl analysis.

In view of the purposes and tasks mentioned above, a bioassay process and the corresponding kit for carrying it out having the characteristics defined in the claims which follow, which constitute an integral part of the present description, constitutes a subject of the invention.

The bioassay according to the invention is based on the discovery of the fact that discontinuous patterns (for example *spots*) of adhesion promoting sites (for example polylysine) for neuronal cells, defined on a continuous substrate having neuronal cell adhesion inhibiting properties (for example agarose), are capable of stabilizing and directing the formation of neuronal extensions and of physical connections depending on the distance between said sites.

Within the scope of the invention, it has further been discovered that there exists between the adhesion sites (spots) a critical distance which influences the ability of the neurons to sense one another and to extend reciprocal physical connections. This critical distance can vary between neuronal cultures from healthy (wild-type) animals and cultures from animal models of diseases.

On the basis of these observations, the method and kit according to the invention provide for the following operations.
A) Deposition onto a substrate inhibiting the adhesion of neuronal cells of a plurality of mutually spaced spots of an adhesion promoting substance for said neuronal cells; this procedure can be carried out with the use of equipment for the production of commercial nanomatrixes ("nanoarrayers").

The aforesaid substrate is generally deposited onto a flat solid support, typically of glass, which is a material of primary choice for the biosciences because of its ease of integration with standard optical microscopy, on account of its transparency. However, there are no particular restrictions as to the nature of the support.

The substrate inhibiting the adhesion of the neuronal cells is typically formed from a substance forming a hydrogel, such as agarose or polyethylene glycol, which is deposited onto the support, preferably by means of a spin-coating technique, which makes it possible to obtain a uniform, homogeneous and controlled thickness. The thickness of the inhibiting substrate is preferably less than 100 nm, more preferably less than 40 nm. Thicknesses of the order of 10 nm to 20 nm have been found particularly advantageous in the case of use of agarose as substrate, in that in this case a better neuronal vitality has been observed and it has been observed that the adhesion promoting substance (in the present case polylysine, PDL) is not only capable of providing adhesion sites for the neurons, but also allows the growth of a confined network which extends to cover the surface of the model between the spots of that substance.

It is thus advantageous to have available very thin substrates, the lower limit of thickness being generally dictated only by the deposition technique (spin-coating) and by the need to obtain continuous substrates, with uniform and homogeneous characteristics.

The adhesion promoting substance for the neuronal cells is in general a substance of protein nature or a polypeptide with exposed amino groups; polymers containing amino groups, such as for example polyethyleneimine (PEI) or nano materials, such as for example carbon nanotubes, which can act as adhesion sites, can also be used.

The spots in the kit and method of the present invention are individually separated from one another and surrounded by the adhesion inhibiting substrate material, preferably exhibit a maximum size of the order of 5 to 40 µm, more preferably from 5 to 25 µm.
B) The neuronal cells subjected to the assay are plated and allowed to adhere in correspondence to the regions patterned with the adhesion promoting substance, and are then maintained in incubation under culture conditions suitable for favouring the growth of neuritic extensions.

The maximum size of the individual spots is such as to accommodate a single cell body, at least statistically, in the adhesion phase, (although, in the growth phase, the cells may be able to shift and possibly amalgamate with one another).
C) The formation of neuritic extensions between the spots is determined after an appropriate incubation period by detecting the formation of such interconnecting neuritic extensions between individual neuronal cells adhered to respective adjacent spots. The detection of the neuritic extensions can preferably be effected by immunofluorescence, but also by electrical measurements (e.g. measurement of impedance) on circuits embedded in the substrate or by optical methods.

For the purposes of the detection of the formation of neuritic extensions by immunofluorescence, the cells can be fixed (for example with paraformaldehyde) with addition of a blocking solution comprising marker antibodies of tubulin and hence of the cytoskeleton (for example anti-βIIITub) and marker antibodies of the glial cells. The marker antibody of tubulin is important for highlighting the dendrites.and the axons. Transfection or infection techniques could be used for "life" detection by causing expression of the fluorescent proteins in the cells under examination.

The glial cell marker (anti-GFAP) is useful for distinguishing neurons from glia.

The measurement of the connections can include the use of optical techniques (microscopy or integrated with the use of waveguides) or electrical and/or electrochemical techniques such as impedance measurement or functional techniques with microelectrodes. Measurement with microelectrodes would also make it possible to check the functionality of the physical neuritic connection.
D) The bioassay according to the invention thus envisages the determination of the critical or maximum distance between the spots corresponding to which a pair of spaced individual neuronal cells are still capable of forming interconnecting neuritic extensions. The determination of that distance is effected by checking the formation of neuritic extensions between individual neuronal cells adhered to respective spots spaced at different distances.

Since the assay envisages the prearrangement of a plurality of pairs of spots with a step variation in their distance, the critical distance cannot coincide with a maximum distance in the absolute sense.

For this purpose, the device for carrying out the assay, or the corresponding kit, can provide for various embodiments.

There can be provided a kit for carrying out the assay comprising a plurality of supports, each of which exhibits at least one respective pattern, for example with a matrix configuration, of equally spaced adhesion spots, and where each pattern exhibits a step between the spots different compared to the step of the other patterns, for example with a constant increment in step. In this case, the critical distance between the spots is determined by carrying out the assay in parallel or sequentially, under the same conditions, using more supports.

Alternatively, there may be envisaged one single support exhibiting a plurality of patterns, where each pattern is formed from a plurality of spots equally spaced according to a first predetermined step, and exhibits a different equal spacing step, for example in increasing order, compared to the other patterns.

The creation on a support of a plurality of spots according to one or more patterns, for example having a linear progression, each having one step between adjacent spots, increasing or decreasing, in a gradual manner, can also be envisaged. Typically, on the basis of the tests performed, the step or distance between the spots of a defined pattern can lie between 10 µm and 200 µm and, in the case wherein several distinct patterns may be created, for example in a matrix, the step increment between patterns can be varied with increments such as to cover the range indicated above.

The configuration of the patterns is not particularly critical and can comprise patterns with a linear progression (e.g. rectilinear) or, as already indicated, for example in a hexagonal or square matrix.

In the assay according to the invention, once the critical distance corresponding to which the formation of interconnecting neuritic extensions is observed has been determined, the operation of comparison of the aforesaid critical distance, determined for a first type of neuronal cells, with the critical distance determined, under the same conditions, for comparison or reference neuronal cells (for example by comparison between wild-type cells and transgenic cells) can be provided for. The reference cells can be healthy cells.

The terms "plurality of spots", "plurality of pairs of spots" and "plurality of patterns" used in the present description preferably involve a number greater than 2, for example 3, 4, 5 ... 10.

Application examples of the bioassay
1. Quantification of alterations in the growth of the neuritic extensions of neuronal cells in culture and comparison between preparations from wild-type animals and from transgenic animals (e.g. mouse).
   **Advantage:**
      A. The bioassay makes it possible to quantify an alteration in the neuritic extension without the need to visualize individual neurons (as required using conventional morphological analysis techniques),
      B. The bioassay can be used as a rapid pre-screening method before carrying out detailed morphological analyses.
         **Example of use:** Neuronal cells (primary or cell lines) are deposited onto the substrates functionalized with an arrangement of the spots which enables them to be read by microscopy of the distance between spots which is (or is not) reached by the neuritic extensions. Such samples are prepared either with wild-type neurons or with neurons from genetically modified animals. The samples are grown for some days (from 1 to 20) in an incubator with appropriate ambient conditions. By optical (e.g. fluorescence) microscopy, for example the maximum distance reached by the neuritic extensions is quantified and that distance is compared with the result obtained between the different typologies of neuronal cells cultured.
2. Quantification of alterations in the growth of the neuritic extensions of human neuronal cells in culture derived from non-embryonic stem cells by cellular reprogramming (e.g. Human iPSC-Derived neurons) and comparison between preparations from healthy and diseased subjects.
   **Advantage:**
      Besides the advantages cited in item 1, in the field of HiPSC, this methodology can be used to obtain preliminary results to quantify the significance of the reprogrammed cell sample from human subjects.
3. Quantification of alterations in the growth of the neuritic extensions of neuronal cells in culture (from animal or human models by reprogramming) induced by pharmacological and/or toxicological substances.
   **Advantage:**
      Besides the advantages cited in item 1, in this application, the bioassay makes it possible to assess the effect of a chemical product, and in a particular manner of the action of recovery of a normal neuritic extension induced by a drug or of the alteration induced by a toxicological product (i.e. chemical or nanoparticle).
         **Example of use:** The assay can be applied but acting pharmacologically (e.g. single administration or constant administration) on a group of samples to assess the differences in the extent of the neuritic growth between neuronal cells of the same origin, but under the action of exogenous agents.

      The scope of the invention does not include the use of human embryonic stem cells.

Further characteristics of the process and of the device or kit for carrying out the assay will follow from the practical examples which follow.

In the appended diagrams:

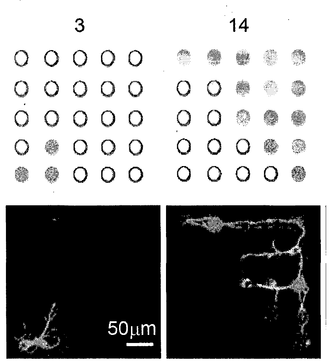

- fig.1a shows immunofluorescence images obtained on three different neuronal after 21 days in vitro (DIV) on identical matrices wherein spots of PDL (0.5 mg/mL) consisting of 5x5 spots separated by a step of 50 µm in the x and y directions are created;

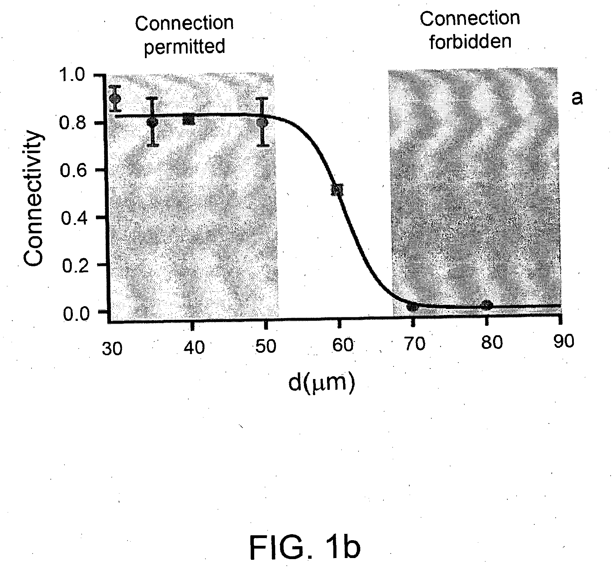

- fig.1b is a diagram which illustrates the empirical parameter of connectivity as a function of the distance between the spots of PDL for a distance between the spots less than 50 µm, corresponding to which a connection is observed, and for a distance greater than 70 µm, corresponding to which the connection is prevented;

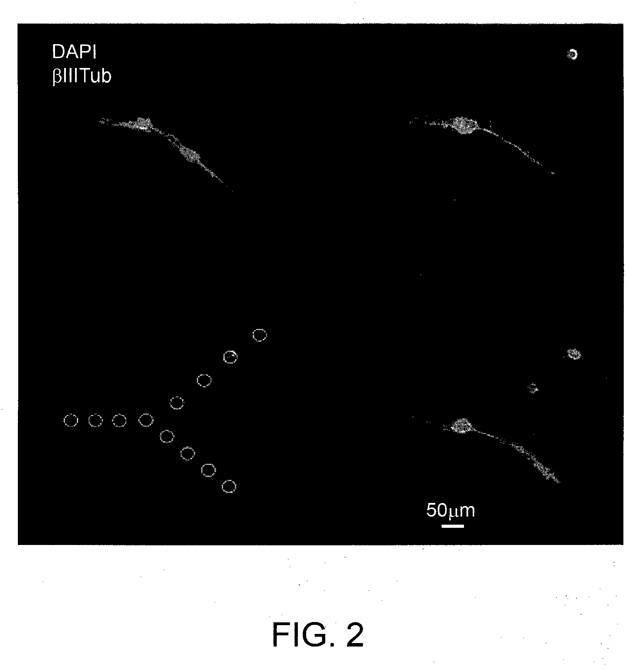

- fig.2 is an immunofluorescence image which illustrates a series of Y-shaped patterns of spots of PDL separated by spaces, under neuronal culture conditions, which demonstrates selective neuronal growth only in the arm of the Y pattern turned towards the bottom, in which the spots of PDL are separated by a distance less than 60 µm, while no connection is found for the upper arm, with a distance between the spots greater than 80 µm.

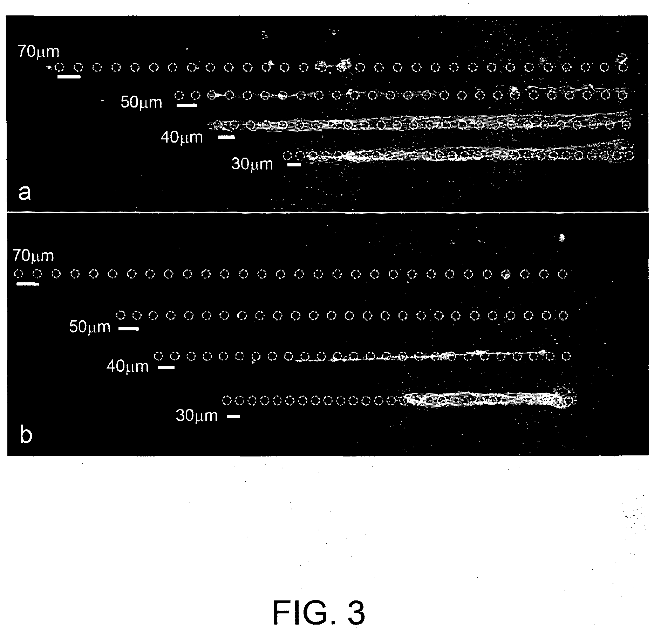

- fig.3 is an immunofluorescence image which illustrates the comparison between the growth and connectivity of hippocampal neurons from wild type mouse and from genetically modified mouse on substrates decorated with identical patterns of spots of adhesion protein. While the neurons from wild type mouse grow branchings which connect spots separated by up to 50 µm, the neurons from genetically modified mouse connect spots separated by distances up to 40 µm.

### Example 1: Preparation of the device

For the preparation of the device, a layer of agarose gel is deposited onto a glass support, for example a glass slide. A 0.15% w/w aqueous solution of agarose (Sigma) is then prepared by dissolving the agarose powder in Milli-Q water brought to the boiling point in a microwave oven. The solution is prepared immediately before use and kept on a hotplate at T = 80°C to keep the agarose in the liquid state. The deposition of agarose with a controlled thickness is obtained by spin-coating (800 rpm, 30 s). The glass slides are pretreated for 20 minutes in a UV/ozone chamber to make the surface hydrophilic and facilitate the diffusion of agarose. The agarose layer is allowed to dry in air at ambient temperature for at least 6 hours. The thickness of the resulting layer was measured with a profilometer. The thin layers obtained by spin coating have a thickness of about 10 nm.

After the preparation of the agarose gel layer, the adhesion proteins are deposited onto it by means of the nanoarrayer. The protein selected is poly-D-lysine (PDL), MW 70,000 (Sigma). The PDL is dissolved in Milli-Q water at a concentration of 1 mg/mL; aliquots of 25 microlitres are frozen and kept at -20°C. One aliquot is diluted with Milli-Q water to give a final concentration of 0.3, 0.5 or 0.7 mg/mL. To enable the deposition of drops of small volume, a small quantity of glycerol (5% w/w) is added to the solutions.

Microdispensing of the proteins: the matrices of microdrops of PDL are produced by the use of the nanoarrayer NanoEnabler (Bioforce), equipped with SPT C30 S cantilevers. The relative humidity in the chamber is maintained at 50 ± 5%. The contact force and the withdrawal distance are 0.002 nN and 40 µm, respectively.

To check the morphology of the spots deposited under dry and wet conditions, a fluorescence-labelled (poly-L-Lysine) PLL (0.5 mg/mL) was used. The comparison of spots of a sample dried in air with the same spots of a sample stored in a saline aqueous environment for one week did not show any difference in the size and morphology of the spot with regard to the use of PDL. Moreover, on the wet sample no signal of diffusion of PPL between the spots was observed and the zone of deposition of the PLL remains localized and stable in terms of dimensions under moist conditions.

The surface of the device was characterized by means of atomic force microscopy (AFM) techniques with the use of an MFP-3D (Asylum Research, USA). From the analyses performed it emerges that the agarose regions and the spots of PDL exhibit similar roughness, independently of the concentration of PDL, indicating that the PDL released by the droplets of solution is completely absorbed by the porous matrix of agarose. To assess the effect of the different concentration of protein, the ratio between the contact rigidity assessed on the spots of PDL (K_{PDL}) and that measured on the surrounding agarose (K_{agar}), which gives the stiffening coefficient S=K_{PDL}/K_{agar}, was measured. The results show that for PDL at 0.1 mg/mL the stiffening effect is very close to the sensitivity threshold of the detection measurement; for an intermediate concentration of 0.3 mg/mL, S increases rapidly, until it almost doubles its value, and finally reaches a plateau for the higher concentrations (0.5 and 0.7 mg/mL). On these substrates, it is possible to carry out standard cell cultures and fixing protocols, if necessary coupled with fluorescence labelling to facilitate the microscopic analysis of the geometry of the resulting cell culture.

After the deposition of the protein, the substrates were sterilized with UV radiation for 30 minutes for the next phase of preparation of the culture of neuronal cells.

### Example 2: Performance of the assay

Experiments were performed in which the distance between the spots was varied in a range of 20-120 µm and different matrix geometries were used. In particular, square matrices of patterns of PDL consisting of 5x5 spots (fig. 1a) were prepared and used as substrate for primary hippocampal cell cultures. The cultures were obtained from cortexes of Sprague-Dawley rats on the eighteenth day of gestation. Embryonic rat cerebral cortexes were sectioned and dissociated. The resulting tissue was resuspended in Neurobasal medium with addition of 2% B-27 and 1% Glutamax-I (Invitrogen, Carlsbad, CA, USA), and the cells were plated onto agarose/PDL at a density of 70 cells/mm². The cultures were kept in a 5% CO₂ incubator at 37°C and taken out only to change the culture medium. To sustain the growth of the low density preparations, cultures were prepared on glass slides in multiwell plates (Corning, Lowell, MA, USA) containing culture medium. Each well was coated with 0.1 mg/mL of PDL and drops of paraffin were used to hold the glass slides. The bottom of the well was seeded with hippocampal cultures with a density of 150 cells/mm². After the seeding, the glass slides were transferred into the wells with sterile forceps and placed on the drops of paraffin with the cell culture side tuned towards the culture medium. The culture medium was changed weekly.

The cells were fixed with paraformaldehyde (PFA). The fixed samples were permeabilized with 0.1% (v/v) Triton X-100 in 1X PBS for 15 mins. A blocking solution (1X PBS, 1% BSA, 5% FBS) was added for 45 minutes at ambient temperature to block nonspecific reactions. The samples were incubated for 3 hours at ambient temperature with the primary monoclonal antibodies (mAb) rabbit anti-mouse β IIITub and anti-GFAP (Millipore, Billerica, MA, USA) diluted in blocking solution. Next, the samples were washed and incubated with a secondary antibody of goat anti-rabbit labelled with Alexa488 or with monoclonal antibody goat anti-mouse labelled with Alexa546 (Invitrogen, Carlsbad, CA, USA) for 45 minutes at ambient temperature. The samples were then mounted using the antifade reagent Prolong containing DAPI (Invitrogen, Carlsbad, CA, USA) and stored at 4°C. The samples were visualized by broad field fluorescence microscopy using the imaging system Neurolucida (Bioscience mbf, Williston, VT, USA) mounted on a BX51 Olympus microscope. The images were analyzed with ImageJ and processed with Adobe Photoshop CS3 Suite (Adobe, San Jose, CA, USA).

The morphology of neural networks developed at 14 and 21 DIV was analyzed. From the immunofluorescence images it was confirmed that on substrates with a thin layer of agarose of 10-20 nm good neuronal vitality is observed and the drops of PDL deposited, as well as providing adhesion sites for the neurons, confine the neuronal growth on the surface of the model. So as to have a reference parameter for comparing different degrees of development of the networks on the bioprinted surfaces, the number of spots in which a physical connection between the neurons took place (fig. 1a) was analyzed. The spots covered with neurons which do not display extensions towards the neurons present on other spots were not considered as connected. In this manner, a ratio of connected spots / total spots is obtained which is compared for the different steps used to create the matrixes. A step is defined as the theoretical centre-centre distance between adjacent spots. All the data were analyzed using OrginPro (OriginLab Corp, Northampton, MA, USA). The statistical significance on the connectivity values was determined by the ANOVA test.

In order to obtain information on the bio-patterning conditions which facilitate or compromise the development of physical connections of neurons between the spots of PDL, the geometric constraints of the pattern were varied and the effects on the inter-spot neuronal connectivity were investigated. In particular, on progressively increasing the distance between the adhesion spots, a limit in the ability of the neurons to extend the connections between the spots was observed. To quantify these experimental data, an empirical connectivity parameter was introduced. This was necessary since the networks were extending themselves differently to cover the patterned surface, i.e. by connecting a different number of adhesion points in the matrix. For example, fig.la shows three neuronal patterns which represent three different development situations, all at 21 DIV and on identical patterns of PDL. In order to quantify these results and to relate them to the distance between the points, an empirical connectivity parameter c was defined as the number of adjacent adhesion spots continuously connected by neurites in a matrix, normalized for the total number of spots in the matrix itself. For example, the upper panel in fig. 1a shows the connectivity values associated with each of the matrices illustrated.

The statistical analysis was performed considering the case of a favourable interface, that is a layer of agarose of thickness t<20 nm, considering a connectivity threshold c>0.25. The results of the mean connectivity value over the population of the matrix which satisfy the criteria are plotted as a function of the inter-spot distance as in fig.1b). The analysis was performed on a number n of samples which range from a minimum of n=48 (step d=30 µm) to a maximum of n=120 (step d=40, 50, 60 and 70 µm) on samples belonging to 15 different primary neuronal cultures deriving from different animals. From fig.1b it clearly emerges that a separation between the spots of less than 50 µm allows neuronal connectivity, while on increasing the distance beyond 70 µm the connection between the neurons is compromised. Between these two situations, a region of uncertainty is observed. In order to demonstrate that these interfaces at two levels can be designed so as to direct the development of neuronal extensions, neuronal cultures were cultivated on simple Y-shaped patterns, with non-homogeneous arrangement of spots of PDL. On the upper arm, the spots are at a distance which exceeds the connectivity distance threshold, while on the lower arm the spots are at a distance less than the threshold. Representative images of the neurons cultivated on these structures are shown in fig. 2.

As expected, in all the Y-shaped models, the neurites develop on the lower arm, connecting spots with a step less than the connectivity distance threshold. Moreover, when they adhere on the spots of PDL of the upper arm, the neurons do not exhibit neuritic extensions and do not survive to 21 DIV.

The bioassay according to the invention can be implemented by providing a device or a kit which comprises one or more solid supports on which there are created one or more patterns of adhesion spots for neuronal cells, according to arrangements previously described and defined in the appended claims, onto which, during performance of the bioassay, the neuronal cells subjected to the assay can be adhered.

The kit can if necessary comprise reagents for detection by immunoassay, particularly by immunofluorescence, of the interconnecting neuronal extensions between the spots formed after an incubation period.

The results obtained according to the invention indicate that the use of the bioassay based on matrixes or patterns of adhered neuronal cells represents a facilitated and simplified experimental model in comparison with in vivo studies, for contributing to the study of functional properties dependent on topology, such as plasticity, regeneration and dynamics of the networks. In current research in the sector, systems of models based on transgenic animals are used in order to investigate such alterations. Since cerebral diseases, such as epilepsy, autism or Alzheimer's disease, are known to be associated with variations in connectivity, and it being known that neuropathologies can have an effect on the neuronal morphologies and on the capacity of the neuronal populations to establish physical connections, the bioassay which is the subject of the invention is capable of providing useful information for the diagnosis of neurodegenerative diseases.

For this purpose, a test was performed wherein neural cultures from wild-type animals and from genetically modified animals and carriers of DiGeorge syndrome were compared. This disease is known to introduce dysfunctionalities in neuronal growth and in particular in the development of the dendritic branchings. The cells were plated and grown up to 14 DIV on substrates with an agarose coating and decorated with patterns of poly-D-lysine according to the technique described; the preselected patterns were series of 5 parallel lines of equidistant spots; each line contained up to 30 spots and the distances between spots were different, in particular steadily increasing, from line to line and equalled 30 µm, 40 µm, 50 µm and 70 µm.

Fig.3 shows the immunofluorescence images obtained from the neurons belonging to the two types of animals on two typical identical patterns. The neurons belonging to the wild-type mice (Fig.3a) grow dendrites and connect spots until they cover distances of 50 µm. The branchings of the neurons of the genetically modified animals manage only to cover distances equal to 40 µm (Fig. 3b).

In one embodiment of the bioassay, if necessary, electrodes corresponding to the spots could be inserted with the aim of quantifying not only the connectivity, but also the functional activity of the neuronal connections which have been formed.

## Claims

1. Bioassay process for the determination of alterations in neuronal connectivity and/or morphology, **characterized in that** it comprises the operations of:
- identifying the critical distance corresponding to which a pair of spaced single neuronal cells subjected to the assay is still able to form reciprocally interconnecting neuritic extensions, after a predetermined incubation period under conditions which favour the formation of such neuritic extensions,
wherein each of said single neuronal cells is adhered to a respective spot of a substance promoting the adhesion of said neuronal cells, said spots being deposited, spaced relative to one another, onto a substrate inhibiting the adhesion of neuronal cells, each of said spots being surrounded by said adhesion inhibiting substrate, and wherein the spots form at least one pattern or a plurality of patterns, each formed by a plurality of separate spots such that said at least one pattern is formed by spots spaced by a progressively increasing step, or each of said plurality of patterns is formed by spots equidistant to each other wherein each pattern exhibits a step between the spots different from the step of the other patterns, and
- comparing the critical distance identified with the critical distance determined, under the same conditions, for reference cells,
wherein a decrease in said critical distance for the neuronal cells subjected to the assay, compared to the critical distance for the reference cells, is indicative of an alteration in the neuronal connectivity and/or morphology.

2. A process according to Claim 1, **characterized in that** it comprises the operations of:
a) producing on a substrate inhibiting the adhesion of neuronal cells a plurality of pairs of spots of a substance promoting the adhesion of said neuronal cells, wherein each of said spots is separate and distinct from the other spots and wherein at least two of said pairs exhibit a different distance between the spots;
b) plating and allowing to adhere correspondingly to each of said spots a single neuronal cell subjected to the assay;
c) detecting, after said predetermined incubation period, the possible formation of neuritic extensions interconnecting adjacent neuronal cells, adherent to the spots of said pairs; and
d) determining said critical distance as the maximum distance between the spots of said pairs corresponding to which the formation of the interconnecting neuritic extensions occurs, said critical distance being determined by comparing the neuritic extension behaviour between pairs of spots at different distances.

3. A process according to claim 1 or 2, **characterised in that** said spots of adhesion promoting substance are deposited on said substrate in accordance with at least one pattern, having an increasing distance step between successive spots.

4. A process according to Claim 1 or 2, wherein a plurality of patterns is deposited onto the aforesaid substrate, each comprising a plurality of equally spaced spots, wherein each pattern exhibits an equal spacing step different from the equal spacing step of the other patterns.

5. A process according to one of Claims 2 to 4, **characterised in that** it comprises the step of repeating the operations a), b) and c), under the same conditions, with the use of a substrate exhibiting at least one pattern of equally spaced spots with a decrease or increase, in each repetition, of the equal spacing step between said spots, in order to determine the critical distance corresponding to which the formation of said extensions is detected or, respectively, not detected.

6. A process according to any one of the previous claims, **characterised in that** the distance between the spots of said pairs is in the range between 5 µm and 200 µm.

7. A process according to any one of the previous claims, **characterised in that** the adhesion inhibiting substance is a layer of agarose or polyethylene glycol having a thickness less than 40 nm.

8. A process according to any one of the previous claims, wherein said adhesion promoting substance is a polypeptide having exposed amino groups, or a polymer including amino groups, or nanoparticles that provide sites for cell adhesion.

9. A process according to any one of the previous claims, wherein the step of detecting the formation of said neuritic extensions is carried out by immunofluorescence assay or assay based on optical or electrical methods, such as impedance measurements on electrodes embedded in the substrate or optics embedded in the substrate.

10. A process according to any one of the previous claims, applied in sequence or in parallel for the determination of the critical interconnecting distance of neuronal cells of a wild-type animal and neuronal cells of the same animal genetically modified.

11. A process according to any one of Claims 1 to 9 for the determination of alterations in the growth of neuritic extensions of human neuronal cells in culture derived from non-embryonic stem cells obtained by cellular reprogramming.

12. A process according to any one of Claims 1 to 10 for determination of the effect of an exogenous agent such as a drug or a toxicological agent on neuronal cells comprising the comparison between the critical interconnecting distance between neuronal cells subjected or not subjected to the action of said agent.

13. Kit for carrying out a process according to any one of Claims 1 to 12, **characterised in that** it comprises one or more solid supports, comprising at least one pattern or a plurality of patterns, each formed by a plurality of separate spots of a substance promoting the adhesion of neuronal cells, deposited on a substrate of a substance inhibiting the adhesion of said neuronal cells, said adhesion-inhibiting substance being deposited as a coating of said support and wherein at least one pattern is formed by spots spaced by a progressively increasing step, or each of said plurality of patterns is formed by spots equidistant to each other wherein each pattern exhibits a step between the spots different from the step of the other patterns and optionally reagents for the detection by immunoassay of interconnecting neuritic extensions between said spots and wherein said spots exhibit a maximum size such as to permit on average the adhesion of a single neuronal cell to each of said spots, and wherein each of said spot is surrounded by said adhesion-inhibiting substance.

14. Kit according to claim 13, wherein said substrate inhibiting the adhesion is a layer of agarose or polyethylene glycol, preferably having a thickness less than 40 nm.

15. Kit according to claim 13 or 14, wherein said adhesion promoting substance is a polypeptide having exposed amino groups or a polymer including amino groups.

## Patentansprüche

1. Bioassay-Verfahren zur Bestimmung von Veränderungen in der neuronalen Konnektivität und/oder -Morphologie, **dadurch gekennzeichnet, dass** es die folgenden Abläufe aufweist:
- Ermitteln des kritischen Abstands, der dem entspricht, bei dem ein Paar von beabstandeten einzelnen Nervenzellen, die dem Assay unterzogen werden, noch in der Lage ist, wechselseitig verbindende neuritische Fortsätze auszubilden, nach einer vorgegebenen Inkubationszeit unter Bedingungen, die die Ausbildung solcher neuritischer Fortsätze begünstigen,
wobei jede der einzelnen Nervenzellen an einer entsprechenden Stelle einer das Anhaften der Nervenzellen begünstigenden Substanz angebunden wird, wobei die Stellen voneinander beabstandet auf einem das Anhaften von Nervenzellen hemmenden Substrat angeordnet sind, jede Stelle von dem das Anhaften hemmenden Substrat umgeben ist, und wobei die Stellen mindestens ein Muster oder eine Vielzahl von Mustern bilden, die jeweils von einer Mehrzahl getrennter Stellen gebildet werden, so dass das mindestens eine Muster von Stellen gebildet wird, die in einer zunehmend ansteigenden Schrittweite voneinander beabstandet sind, oder jedes der Mehrzahl von Mustern durch äquidistante Stellen gebildet wird, wobei jedes Muster eine Schrittweite zwischen den Stellen aufweist, die sich von der Schrittweite der anderen Muster unterscheidet, und
- Vergleichen des ermittelten kritischen Abstands mit dem unter denselben Bedingungen für Vergleichszellen bestimmten kritischen Abstand, wobei eine Abnahme des kritischen Abstands für die dem Assay unterworfenen Nervenzellen im Vergleich zu dem kritischen Abstand für die Vergleichszellen einen Hinweis auf eine Veränderung in der neuronalen Konnektivität und/oder -Morphologie ist.

2. Verfahren nach Anspruch 1, **dadurch gekennzeichnet, dass** es die folgenden Abläufe aufweist:
a) Erstellen von einer Mehrzahl von Paaren von Stellen aus einer das Anhaften der Nervenzellen fördernden Substanz auf einem das Anhaften von Nervenzellen hemmenden Substrat, wobei jede der Stellen von den anderen Stellen getrennt und unterscheidbar ist und wobei mindestens zwei der Paare einen unterschiedlichen Abstand zwischen den Stellen aufweisen;
b) Auftragen und Ermöglichen des Anhaftens einer einzelnen dem Assay unterworfenen Nervenzelle an jeweils jeder der Stellen;
c) Ermitteln der möglichen Ausbildung neuritischer Fortsätze, die an den Stellen des Paares anhaftende benachbarte Nervenzellen miteinander verbinden, nach vorgegebener Inkubationszeit; und
d) Bestimmen des kritischen Abstands als dem maximalen Abstand zwischen den Stellen der Paare, die denen entsprechen, zwischen denen die Ausbildung der verbindenden neuritischen Fortsätze auftritt, wobei der kritische Abstand durch Vergleich des neuritischen Fortsatzverhaltens zwischen Paaren von Stellen mit verschiedenen Abständen bestimmt wird.

3. Verfahren nach Anspruch 1 oder 2, **dadurch gekennzeichnet, dass** die Stellen der das Anhaften fördernden Substanz gemäß mindestens einem Muster, das zwischen aufeinanderfolgenden Stellen einen zunehmenden Abstandsschritt aufweist, auf dem Substrat angeordnet sind.

4. Verfahren nach Anspruch 1 oder 2, wobei eine Mehrzahl von Mustern auf dem zuvor genannten Substrat aufgebracht wird, von denen jedes eine Mehrzahl von gleichmäßig beabstandeten Stellen aufweist, wobei jedes Muster einen gleichen Abstandsschritt aufweist, der von dem gleichen Abstandsschritt der anderen Muster verschieden ist.

5. Verfahren nach einem der Ansprüche 2 bis 4, **dadurch gekennzeichnet, dass** es den Schritt des Wiederholens der Abläufe a), b) und c) unter den gleichen Bedingungen unter der Verwendung eines Substrats umfasst, das mindestens ein Muster mit gleich beabstandeten Stellen mit einer Abnahme oder Zunahme des gleichen Abstandsschritts zwischen den Stellen bei jeder Wiederholung aufweist, um den kritischen Abstand zu bestimmen, der dem entspricht, bei dem die Ausbildung der Fortsätze nachgewiesen bzw. nicht nachgewiesen wird.

6. Verfahren nach einem der vorangehenden Ansprüche, **dadurch gekennzeichnet, dass** der Abstand zwischen den Stellen des Paares im Bereich zwischen 5 µm und 200 µm liegt.

7. Verfahren nach einem der vorangehenden Ansprüche, **dadurch gekennzeichnet, dass** die das Anhaften hemmende Substanz eine Schicht aus Agarose oder Polyethylenglykol mit einer Dicke von weniger als 40 nm ist.

8. Verfahren nach einem der vorangehenden Ansprüche, wobei die das Anhaften fördernde Substanz ein Polypeptid mit freiliegenden Amino-Gruppen oder ein Polymer mit Amino-Gruppen, oder Nanopartikeln, die Stellen zur Zellanhaftung bereitstellen, ist.

9. Verfahren nach einem der vorangehenden Ansprüche, wobei der Schritt zum Nachweis der Ausbildung der neuritischen Fortsätze durch einen Immunfluoreszenz-Assay oder durch einen auf optischen oder elektrischen Methoden basierenden Assay ausgeführt wird, wie beispielsweise Impedanzmessungen an in dem Substrat eingebetteten Elektroden oder in dem Substrat eingebetteter Optik.

10. Verfahren nach einem der vorangehenden Ansprüche, das zur Bestimmung des kritischen Verbindungsabstands von Nervenzellen eines Wildtyp-Tieres und von genetisch veränderten Nervenzellen desselben Tieres der Reihe nach oder parallel angewendet wird.

11. Verfahren nach einem der Ansprüche 1 bis 9 zur Bestimmung von Veränderungen im Wachstum neuritischer Fortsätze von menschlichen Nervenzellen in Kulturen, die aus durch zellbasierte Umprogrammierung erhaltenen nicht embryonischen Stammzellen abgeleitet sind.

12. Verfahren nach einem der Ansprüche 1 bis 10 zur Bestimmung der Wirkung eines exogenen Wirkstoffs, wie beispielsweise eines Medikaments oder eines toxikologischen Wirkstoffs auf Nervenzellen, das den Vergleich zwischen dem kritischen Verbindungsabstand zwischen Nervenzellen, die der Aktivität des Wirkstoffs ausgesetzt oder nicht ausgesetzt sind, aufweist.

13. Satz zur Ausführung eines Verfahrens nach einem der Ansprüche 1 bis 12, **dadurch gekennzeichnet, dass** er einen oder mehrere feste Träger aufweist, die mindestens ein Muster oder eine Mehrzahl von Mustern aufweisen, die jeweils durch eine Mehrzahl getrennter Stellen einer das Anhaften von Nervenzellen fördernden Substanz ausgebildet sind und auf einem das Anhaften der Nervenzellen hemmenden Substrat angeordnet sind, wobei die das Anhaften hemmende Substanz als eine Beschichtung des Trägers aufgetragen ist und wobei mindestens ein Muster durch Stellen ausgebildet ist, die mit einer zunehmend ansteigenden Schrittweite voneinander beabstandet sind, oder jedes der Mehrzahl von Mustern durch zueinander äquidistante Stellen ausgebildet ist, wobei jedes Muster eine Schrittweite zwischen den Stellen aufweist, die von der Schrittweite der anderen Muster verschieden ist, und wahlweise Reagenzien zum Nachweis von verbindenden neuritischen Fortsätzen zwischen den Stellen durch einen Immuno-Assay, und wobei die Stellen eine maximale Größe aufweisen, um im Durchschnitt das Anhaften einer einzelnen Nervenzelle an jeder der Stellen zu erlauben, und wobei jede Stelle von der das Anhaften hemmenden Substanz umgeben ist.

14. Satz nach Anspruch 13, wobei das das Anhaften hemmende Substrat eine Schicht aus Agarose oder Polyethylenglykol, vorzugsweise mit einer Dicke von weniger als 40 nm ist.

15. Satz nach Anspruch 13 oder 14, wobei die das Anhaften fördernde Substanz ein Polypeptid mit einer freiliegenden Amino-Gruppe oder ein Polymer mit Amino-Gruppen ist.

## Revendications

1. Procédé d'essai biologique pour la détermination de modifications de connectivité et/ou de morphologie neuronale, **caractérisé en ce qu'**il comprend les opérations consistant à :
- identifier la distance critique à laquelle une paire de cellules neuronales uniques espacées soumises à l'essai qui est encore capable de former des extensions neuritiques réciproquement interconnectées, après une période d'incubation prédéterminée dans des conditions qui favorisent la formation de telles extensions neuritiques,
dans lequel chacune desdites cellules neuronales uniques est collée sur une tache respective d'une substance favorisant l'adhérence desdites cellules neuronales, lesdites taches étant déposées, espacées les unes des autres, sur un substrat inhibant l'adhérence de cellules neuronales, chacune desdites taches étant entourée par ledit substrat inhibant l'adhérence, et dans lequel les taches forment au moins un motif ou une pluralité de motifs, chacun formé par une pluralité de taches séparées de telle sorte que ledit au moins un motif est formé par des taches espacées d'un pas progressivement croissant, ou chacun de ladite pluralité de motifs est formé par des taches équidistantes les unes des autres, dans lequel chaque motif affiche un pas entre les taches différents du pas des autres motifs, et
- comparer la distance critique identifiée avec la distance critique déterminée, dans les mêmes conditions, pour des cellules de référence, dans lequel une diminution de ladite distance critique pour les cellules neuronales soumises à l'essai, en comparaison à la distance critique pour les cellules de référence, est indicative d'une modification de connectivité et/ou de morphologie neuronale.

2. Procédé selon la revendication 1, **caractérisé en ce qu'**il comprend les opérations consistant à :
a) produire sur un substrat inhibant l'adhérence de cellules neuronales une pluralité de paires de taches d'une substance favorisant l'adhérence desdites cellules neuronales, dans lequel chacune desdites taches est séparée et distincte des autres taches et dans lequel au moins deux desdites paires affichent une distance différente entre les taches ;
b) plaquer et laisser adhérer en conséquence à chacune desdites taches une cellule neuronale unique soumise à l'essai ;
c) détecter, après ladite période d'incubation prédéterminée, la formation possible d'extensions neuritiques interconnectant des cellules neuronales adjacentes, adhérentes aux taches desdites paires ; et
d) déterminer ladite distance critique comme la distance maximale entre les taches desdites paires à laquelle se produit la formation des extensions neuritiques d'interconnexion, ladite distance critique étant déterminée en comparant le comportement d'extensions neuritiques entre des paires de taches à différentes distances.

3. Procédé selon la revendication 1 ou 2, **caractérisé en ce que** lesdites taches de substance favorisant l'adhérence sont déposées sur ledit substrat en conformité avec au moins un motif, ayant un pas de distance croissant entre des taches successives.

4. Procédé selon la revendication 1 ou 2, dans lequel une pluralité de motifs est déposée sur le substrat précité, chacun comprenant une pluralité de taches à égale distance, dans lequel chaque motif affiche un pas d'espacement égal différent du pas d'espacement égal des autres motifs.

5. Procédé selon l'une des revendications 2 à 4, **caractérisé en ce qu'**il comprend l'étape consistant à répéter les opérations a), b) et c), dans les mêmes conditions, avec l'utilisation d'un substrat affichant au moins un motif de taches à égale distance avec une diminution ou augmentation, dans chaque répétition, du pas d'espacement égal entre lesdites taches, afin de déterminer la distance critique à laquelle la formation desdites extensions est détectée ou, respectivement, non détectée.

6. Procédé selon l'une quelconque des revendications précédentes, **caractérisé en ce que** la distance entre les taches desdites paires est dans la plage entre 5 µm et 200 µm.

7. Procédé selon l'une quelconque des revendications précédentes, **caractérisé en ce que** la substance inhibant l'adhérence est une couche d'agarose ou de polyéthylène glycol ayant une épaisseur de moins de 40 nm.

8. Procédé selon l'une quelconque des revendications précédentes, dans lequel ladite substance favorisant l'adhérence est un polypeptide ayant des groupes amine exposés, ou un polymère incluant des groupes amine, ou des nanoparticules qui fournissent des sites pour adhérence cellulaire.

9. Procédé selon l'une quelconque des revendications précédentes, dans lequel l'étape consistant à détecter la formation desdites extensions neuritiques est effectuée par un essai d'immunofluorescence ou un essai basé sur des méthodes optiques ou électriques, telles que des mesures d'impédance sur des électrodes noyées dans le substrat ou une optique noyée dans le substrat.

10. Procédé selon l'une quelconque des revendications précédentes, appliqué en séquence ou en parallèle pour la détermination de la distance d'interconnexion critique de cellules neuronales d'un animal de type sauvage et de cellules neuronales du même animal génétiquement modifié.

11. Procédé selon l'une quelconque des revendications 1 à 9, pour la détermination de modifications dans la croissance d'extensions neuritiques de cellules neuronales humaines en culture dérivées de cellules souches non embryonnaires obtenues par reprogrammation cellulaire.

12. Procédé selon l'une quelconque des revendications 1 à 10, pour la détermination de l'effet d'un agent exogène tel qu'un médicament ou un agent toxicologique sur des cellules neuronales comprenant la comparaison entre la distance d'interconnexion critique entre des cellules neuronales soumises ou non soumises à l'action dudit agent.

13. Matériel pour la mise en oeuvre d'un procédé selon l'une quelconque des revendications 1 à 12, **caractérisé en ce qu'**il comprend un ou plusieurs supports solides, comprenant au moins un motif ou une pluralité de motifs, chacun formé par une pluralité de taches séparées d'une substance favorisant l'adhérence de cellules neuronales, déposées sur un substrat d'une substance inhibant l'adhérence desdites cellules neuronales, ladite substance inhibant l'adhérence étant déposée comme un revêtement dudit support et dans lequel au moins un motif est formé par des taches espacées par un pas progressivement croissant, ou chacun de ladite pluralité de motifs est formé par des taches équidistantes les unes avec les autres, dans lequel chaque motif affiche un pas entre les taches différent du pas des autres motifs et facultativement des réactifs pour la détection par essai immunologique d'extensions neuritiques d'interconnexion entre lesdites taches et dans lequel lesdites taches affichent une taille maximale de manière à permettre en moyenne l'adhérence d'une cellule neuronale unique à chacune desdites taches, et dans lequel chacune desdites taches est entourée par ladite substance inhibant l'adhérence.

14. Matériel selon la revendication 13, dans lequel ledit substrat inhibant l'adhérence est une couche d'agarose ou de polyéthylène glycol, de préférence ayant une épaisseur de moins de 40 nm.

15. Matériel selon la revendication 13 ou 14, dans lequel ladite substance favorisant l'adhérence est un polypeptide ayant des groupes amine exposés, ou un polymère incluant des groupes amine.
